# EUROPEAN PATENT SPECIFICATION

(11) **EP 2 209 456 B1**
(45) Date of publication and mention of the grant of the patent: **06.03.2013**
(21) Application number: 08839618.9
(22) Date of filing: 15.10.2008
(51) Int. Cl.: A61J 3/07

(54) **LINKERS FOR MULTIPART DOSAGE FORMS FOR RELEASE OF ONE OR MORE PHARMACEUTICAL COMPOSITIONS, AND THE RESULTING DOSAGE FORMS**
VERBINDUNGSELEMENTE FÜR MEHRTEILIGE DOSIERFORMEN ZUR FREISETZUNG VON EINER ODER MEHREREN PHARMAZEUTISCHEN ZUSAMMENSETZUNGEN UND RESULTIERENDE DOSIERFORMEN
DISPOSITIFS DE LIAISON POUR FORMES POSOLOGIQUES À ÉLÉMENTS MULTIPLES ET PERMETTANT L'ADMINISTRATION D'UNE OU PLUSIEURS COMPOSITIONS PHARMACEUTIQUES, ET FORMES POSOLOGIQUES EN RÉSULTANT

(30) Priority: 15.10.2007 US 960786 P
(43) Date of publication of application: 28.07.2010
(60) Divisional application: 12185031.7
(73) Proprietor: Capsugel Belgium NV, 2880 Bornem (BE)
(72) Inventor: MCALLISTER, Stephen Mark, Essex CM19 5AW (GB)
(74) Representative: Hyden, Martin Douglas
(86) International application number: PCT/EP2008/063853
(87) International publication number: WO 2009/050190

(56) References cited:
- WO-A-01/08666
- WO-A-2004/010978
- WO-A-2005/039474
- US-A- 4 738 724
- US-A- 5 680 946

## Description

### FIELD OF THE INVENTION

This invention relates to pharmaceutical dosage forms and, more particularly, to multipart capsules including a linker unit and one or more connected sub- units for oral dosing.

### BACKGROUND OF THE INVENTION

Various types of pharmaceutical dosage forms are known for oral dosing. Such capsules generally comprise an envelope wall of a pharmaceutically acceptable, e.g. orally ingestible, polymer material such as gelatin, although other materials for capsule walls, e.g. starch and cellulose based polymers are also known. Such capsules generally have soft walls made by forming a film on a capsule former, which is then allowed to dry. Rigid walled capsules made by injection molding are also known; see for example U.S. 4,576,284, U.S. 4,591 ,475, U.S. 4,655,840, U.S. 4,738,724, U.S. 4,738,817, and U.S. 4,790,881 (all to Warner Lambert). These disclose specific constructions of capsules made of gelatin, starch and other polymers, and methods of making them by injection molding of hydrophilic polymer, e.g., water mixtures. U.S. 4,576,284 specifically discloses such capsules provided with a cap which closes the capsule and is formed in situ on the filled capsule by molding. U.S. 4,738,724 discloses a wide range of rigid capsule shapes and parts.

Multi-compartment capsules, including those of the type where each compartment has different drug release characteristics or, for example, contains a different drug substance or formulation, are also known; see for example U.S. 4,738,724 (Warner-Lambert), U.S. 5,672,359 (University of Kentucky), U.S. 5,443,461 (Alza Corp.), WO 9516438 (Cortecs Ltd.), WO 9012567 (Helminthology Inst. ), DE-A- 3727894, BE 900950 (Warner Lambert), FR 2524311 , NL 7610038 (Tapanhony NV), FR 28646 (Pluripharm), and U.S. 3,228,789 (Glassman), U.S. 3,186,910 (Glassman), among others. U.S. 4,738,817, U.S. 3,228,789, and U.S. 3,186,910 each disclose a multicompartment capsule made of a water-plasticized gelatin.

Pharmaceutical dosage forms that comprise a matrix of a solid polymer, in which a drug substance is dispersed, embedded or dissolved as a solid solution are also known. Such matrixes may be formed by an injection molding process. This technology is discussed in Cuff G. and Raouf F., Pharmaceutical Technology, June 1998, p. 96-106. Some specific formulations for such dosage forms are, for example disclosed in U.S. 4,678,516; U.S. 4,806,337; U.S. 4,764,378; U.S. 5,004,601 ; U.S. 5,135,752; U.S. 5,244,668; U.S. 5,139,790; U.S. 5,082,655 among others, in which a polyethylene glycol ("PEG") matrix is used and solid dosage forms are made by injection molding.

See, also for example, WO 01/08666, WO 02/060385, US 2004/0115256, US 2006/0049311 , WO 02/060384, US 2003/0068369, US 2004/0166153, WO 04/010978, US 2006/0057201 , WO 05/009380, US 2005/0175687, WO 05/089726, US 2005/0249807, US 60/968,383, US 61/061 ,275, and the content of PCT/EPOO/07295 entitled "MULTI-COMPONENT PHARMACEUTICAL DOSAGE FORM". WO 01/08666 relates to multi-component pharmaceutical dosage form comprising two or more connected sub-units, particularly for oral dosing.

### SUMMARY OF THE INVENTION

In one aspect of the present invention, a linker for connecting to one or more dosage form units from the group including capsule compartments and closure caps includes a solid drug substance in tablet form, the tablet having a longitudinal axis and being substantially cylindrical with opposed axial end faces. The linker also includes a jacket formed around and radially confining the tablet. The jacket has an outer wall with longitudinal ends, one or both of the jacket ends being open for dispensing the drug substance from the respective end faces. The jacket outer wall further has snap-fit elements located adjacent one or both longitudinal ends. Preferably, the snap-fit elements are selected from circumferential grooves, groove segments, ridges, and ridge segments.

In another aspect of the present invention, a multicomponent dosage form includes a linker unit having a solid drug substance in tablet form disposed within a jacket, the tablet having a longitudinal axis and being substantially cylindrical with opposed longitudinal end faces. The jacket radially confines the tablet and has an outer wall with longitudinal ends and a raised circumferential band between the jacket ends. The jacket outer wall also has a snap-fit element located adjacent one longitudinal end. The multicomponent dosage form also includes at least one dosage form unit selected from the group including capsule compartments and closure caps, the dosage form unit having an open end connected to the one jacket longitudinal end of the linker unit. The dosage form unit has a snap-fit element complementary to and configured to engage the snap-fit element of the one jacket longitudinal end.

It is to be understood that both the foregoing general description and the following detailed description are exemplary and explanatory only and are not restrictive of the invention, as claimed.

The accompanying drawings, which are incorporated in and constitute a part of this specification, illustrate several embodiments of the invention and together with the description, serve to explain the principles of the invention.

### BRIEF DESCRIPTION OF THE DRAWINGS

The invention will now be described by way of example with reference to:

Fig. 1 which is a perspective view of a linker made in accordance with one aspect of the present invention;

Fig. 2 is a cross section of a preformed tablet component of the linker depicted in Fig. 1 ;

Fig. 3 shows various perspective views of the preformed tablet component shown in Fig. 2;

Fig. 4 is a cross sectional view of the linker shown in Fig. 1 and having a closing wall at one end;

Fig. 5 shows various perspective views of the linker shown in Fig. 4;

Fig. 6 is an exploded view of a multipart dosage form utilizing the linker shown in Figs. 1 and 2;

Fig. 7 is a cross sectional view of an assembled dosage form including the linker shown in Fig. 1 ;

Fig. 8 is an enlarged view of detail A in Fig. 7;

Fig. 9 shows an alternative construction of the groove-type snap-fit element shown in Fig. 8.

### DETAILED DESCRIPTION

Reference will now be made in detail to the present embodiments of the invention, examples of which are illustrated in the accompanying drawings.

In accordance with one aspect of the present invention, a linker is disclosed for connecting with one or more dosage form units from the group including capsule compartments and closure caps, with the linker holding a drug substance. Specifically, the linker includes a solid drug substance in tablet form, the tablet having a longitudinal axis and being substantially cylindrical with opposed axial end faces. The linker further includes a jacket formed around and radially confining the tablet, the jacket having an outer wall with longitudinal ends, one or both of the jacket ends being opened for dispensing the drug substance from the respective end face. The linker still further includes the jacket outer wall a having snap-fit element adjacent at least one jacket longitudinal end, the snap-fit element preferably being selected from circumferential grooves, groove segments, ridges, and ridge segments.

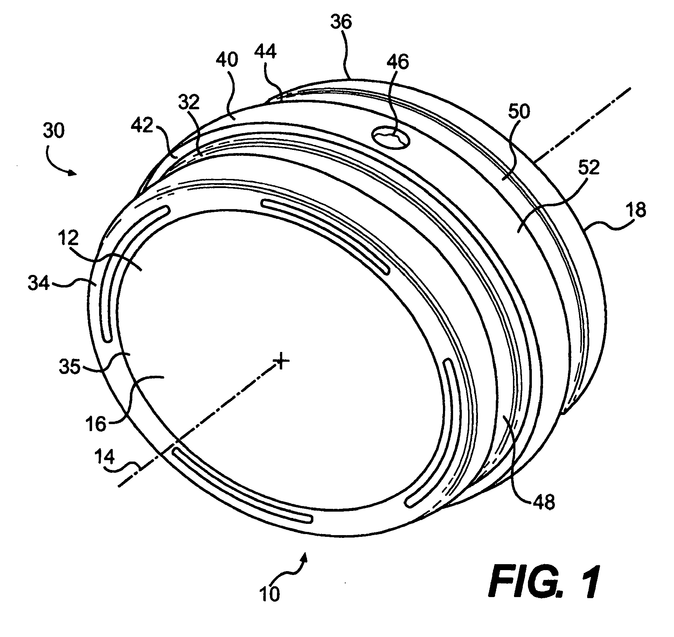

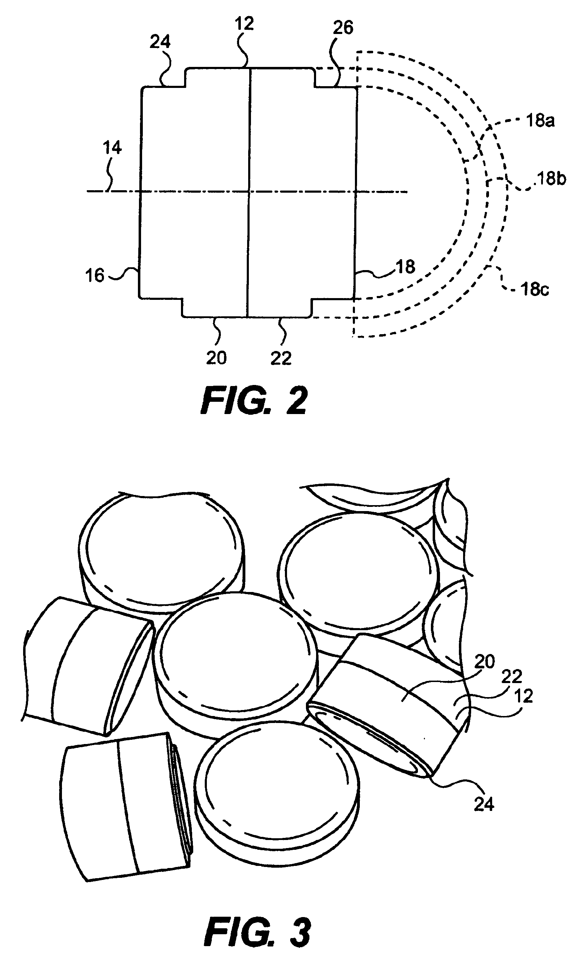

As embodied herein and with initial reference to Figs. 1-4, linker 10 includes tablet 12, which may be substantially cylindrical, having an axis 14 and closed axial end faces 16 and 18. End faces 16 and 18 may be generally planar and perpendicular to axis 14, or one or both may be shaped to extend axially, for reasons to be discussed below, such as rounded end face 18a, 18b, or 18c depicted by dotted lines in Fig. 2. Tablet 12 preferably is preformed outside linker 10 by processes such as dry compacting, casting, or other process known in the art. Tablet 12 can be composed of a single drug substance or, as shown in Fig. 2, a bi-layer configuration can be used having drug substance parts 20, 22. The drug substances in parts 20 and 22 may differ in composition and/or release characteristics, and can be appropriately indicated as such by coloration to ensure correct assembly into dosage forms, as will be discussed below. Also, it is preferred that tablet 12 includes recessed lands 24, 26 formed around respective perimeters of tablet end faces 16, 18, for securing jacket 30 of linker 10 as will be discussed below.

Drug substances for use in dosage forms suitable for being administered orally to a patient can be included in a linker 10 and, in particular, that can be used in parts 20 and 22, can include any suitable or conventional form, such as, for example, a powder, granules, compact, microcapsules, gel, syrup, or liquid, provided that the capsule portion wall material is sufficiently inert to the liquid content of the latter three forms.

As embodied herein and with continued reference to Figs. 1, 4 and 5, linker 10 includes a jacket 30 with a generally cylindrical outer wall 32 and respective longitudinal ends 34, 36. Jacket 30 may be constructed by injection molding around tablet 12 in order to leave one or both jacket ends 34, 36 open and to expose tablet end face 16 and/or 18 for dispersion and dissolution of the contained drug substance(s) once a connected capsule and/or cap dosage form unit has been breached, such as, for example, by changing shape, form, or structure within a gastro-intestinal environment, e.g., dispersing, dissolving, disintegrating, swelling, being partially or completely soluble, or otherwise changeable when exposed to stomach pH and/or in intestine pH. It is contemplated that jacket 30 may be injection molded around tablet 12 and that tablet 12 may be supported within a mold by, for example, one or more finger or gripper elements extending outward from a mold wall into the interior of the mold and engaging a portion of tablet 12, e.g., engaging the radially outer wall of tablet 12.

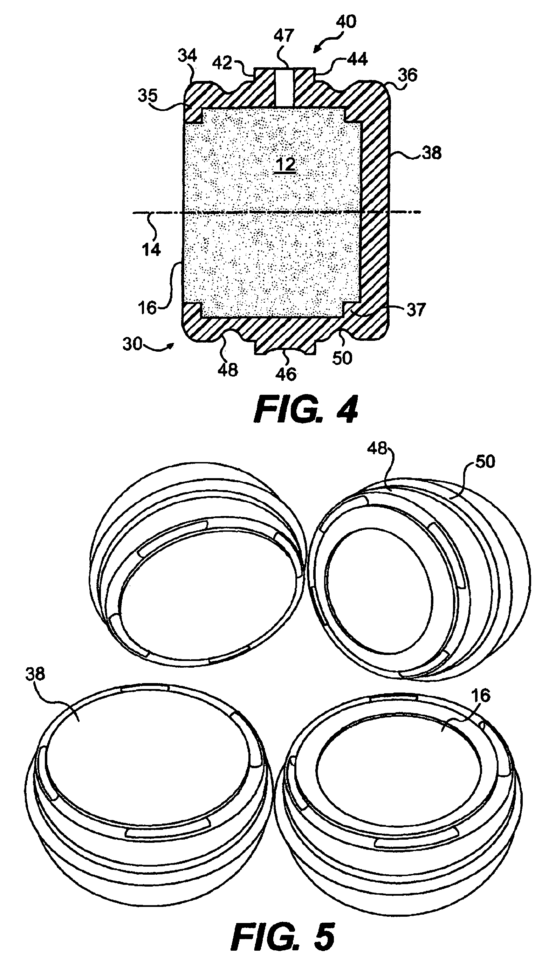

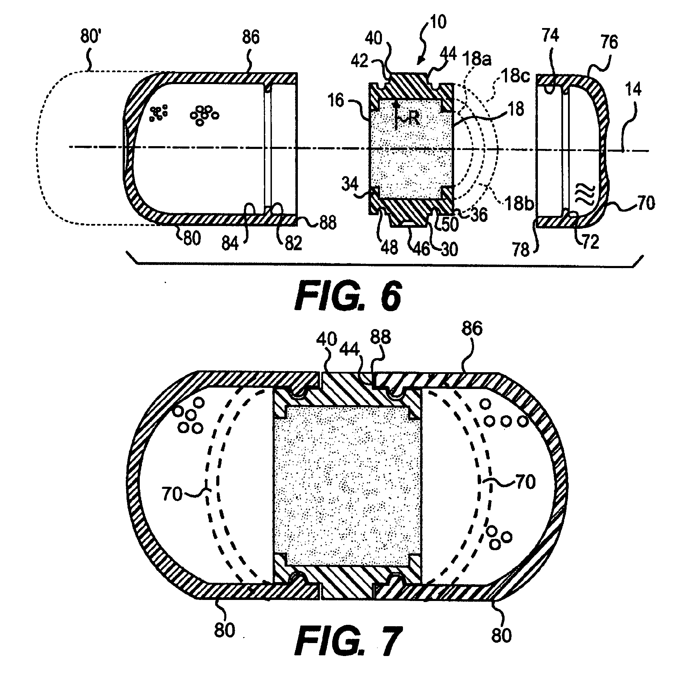

For example, in linker 10, shown in cross-section in Fig. 6, both jacket ends 34, 36 are open to expose tablet end faces 16, 18 for drug substance dissolution and/or dispersion, when cap unit 70 and/or capsule unit 80 are breached. In comparison, Figs. 4 and 5 show a variation in jacket 30 with a solid wall 38 closing off jacket end 36, to substantially prevent the drug substance in tablet 12 from dissolving/dispersing therethrough. Wall 38 can be integrally formed with the remainder of jacket 30 via injection molding. In both variations the injection molded jacket material radially confines a tablet 12 (radial direction being depicted in Fig. 6 as indicated by arrow R) and also axially confines tablet 12 to at least some degree as a result of jacket material flowing into the recess lands 24, 26 of tablet 12 and forming circumferential jacket end edges 35, 37. It is contemplated that a radially confining injection molded jacket includes the jacket surrounding at least a portion and contacting at least a portion of the circumference of the tablet, and may, for example, include the jacket being injection molded around the tablet or other method providing a jacket radially confining the tablet. It is also contemplated that slots 90 may be formed in jacket 30 at one or both of jacket ends 34, 36 due to tablet 12 being supported within a mold by the fingers or grippers (discussed above) during injection molding and which may aid in dissolution/dispersion of the tablet. See Figs. 1 and 5. Although four slots are illustrated in jacket end 34 (see Fig. 1 ), it is contemplated that any number of slots may be formed.

As further embodied herein, and referring again to Fig. 1, jacket 30 includes a raised band 40 circumferential Iy formed on the periphery of outer jacket wall 32, preferably midway between longitudinal ends 34 and 36. Band 40 includes opposed side surfaces 42, 44 configured for abutting contact with the wall ends of dosage form units, e.g., cap unit 70 and/or capsule unit 80, interconnected with linker 10. As depicted in Figs. 1 and 4-6, side surfaces 42, 44 may be essentially perpendicular to axis 14 and raised band 40 also can have one or more concave depressions 46 to accommodate an injection molding feedgate. Also, as shown in Fig. 4, raised band 40 may have one or more radially directed apertures 47 to provide a direct path for controlled, relatively early dispersion of the drug substance in tablet 12, prior to breach or dissolution of any cap or capsule covering exposed tablet end faces, such as end face 16 in Fig. 4. Apertures 47 may be sealed with a rapidly dissolving thin film or coating (not shown) to prevent contamination of the drug substance of tablet 12. Such a film or coating may be made from any Phama-acceptable polymer suitable for film coating.

As further embodied herein, jacket 30 includes snap-fit elements 48, 50 formed on outer surface 52 of jacket wall 32 between raised band 40 and respective jacket longitudinals ends 34, 36. As shown in Figs. 1 and 4, both snap-fit elements 48, 50 are circumferential grooves configured and dimensioned to engage complementary circumferential ridge or "bead" elements on the inner surfaces of the respective cap unit or capsule unit. For example, cap unit 70 shown in Fig. 6 has a continuous circumferential ridge or bead 72 formed on inner surface 74 of outer wall 76 adjacent wall end 78. Ridge 72 is configured to engage groove 50 in linker 10 by a "snap-fit inter-connection." By snap-fit inter-connection, it is meant that the resiliency of cap unit 70 expands to allow ridge 72 to pass over a jacket surface 52 and subsequently contracts to allow ridge 72 to engage or "snap" into groove 50.

Similarly, and as shown in Fig. 6, capsule unit 80 has a circumferential ridge 82 on inner surface 84 of capsule unit wall 86 adjacent wall end 88 configured and dimensioned to be complementary to groove 48 of linker 10 provide a snap-fit inter connection.

Referring to Figs. 6 and 7, it can be seen that linker 10 may make possible multiple dosage form configurations interconnected with a snap-fit connections, including two capsule units 80 joined by linker 10 (see Fig. 7); two cap units 70 joined by linker 10 (see Fig. 7 shown as dashed); or a cap unit 70 joined by linker 10 to a capsule unit 80 (see Fig. 6). However, linker 10 can be used with only a single joined capsule, such as capsule 80 in the embodiment depicted in Fig. 6 but without cap 70, such as if the linker 30 is configured to have a solid end wall, such as end wall 38 in the Fig. 4 embodiment. Moreover, if dissolution/dispersion of an increased quantity of the solid drug in linker 30 is desired, the dosage form in Fig. 6 may be used without end cap 70 with the end face 18 drug material extended axially past linker end. In such a variation, the tablet end face 18 also may be shaped to provide easier swallowing such as with a rounded, extended face such as 18a depicted with dashed line in Fig. 6. Other configurations of an extended tablet end face that may be desirable include those depicted in Fig. 6 as 18b and 18c. Extended end face 18b may be achieved by omitting recess 26 in tablet 12 (see Fig. 2) and jacket end edge 37 (see Fig. 4) and tapering the jacket and edge 36 to provide a smoother dosage form end. A "mushroom" shaped extended end face configuration in 18c is an alternative way to achieve a smooth dosage form end. Also, "exposed" tablet end face 18a, 18b, or 18c may be covered with a thin rapidly dissolving film or coating (not shown) to prevent contamination of the drug substance of tablet 12. Such a film or coating may be made from any Phama- acceptable polymer suitable for film coating.

Further, in each of the above-discussed variants where linker end 36 is to be left uncapped, a snap-fit connection adjacent linker end 36 such as groove 50 may not be needed and band 40 tapered axially, thereby possibly simplifying the construction and manufacture of the linker. However, if linker 30 is constructed without a jacket end edge, such as for extended tablet end faces 18b and 18c, then it may be desirable to use a film covering (not shown) at least over the extended tablet face and adjacent linker end 36 to help axially constrain tablet 18.

Fig. 6 also depicts the use of capsules of varying length, e.g., a longer length capsule 80' shown as dashed, making possible asymmetric dosage forms. Moreover, while Figs. 1 and 4-7 show substantially symmetrical linkers having outer wall diameters at longitudinal ends 34 and 36 substantially equal and configured for attaching capsule and/or cap units of essentially the same diameter, variations in linker 10 having different jacket wall diameters at ends 34 and 36 are contemplated to interconnect capsule units and/or cap units of different diameters, thereby making possible a dosage form asymmetric in width.

Moreover, it is contemplated that the dimensions of the capsules, end caps, and linkers along with the respective snap-fit elements, e.g., cap unit 70 and linker 30, including respective ridge 72 and groove 50 in Fig. 6, may be adjusted to achieve any degree of snap-fit and that the snap-fit is preferably a relatively permanent, locking interconnection therebetween. That is, although the parts could be separated with sufficient force, such separation is not intended in the normal course of utilization of the dosage forms by a consumer. It is also contemplated that the position of the groove and ridge elements could be reversed, and that complementary ridge segments and groove segments could be used instead of circumferentially continuous members. Also, although ridge segments could be used with continuous grooves, leakage of the contained drug substances might possibly occur. Further, although the complementary grooves and ridges/"beads" shown in Figs. 1 and 4-7 are generally rounded or circular in axial cross section, other shapes such as, for example, wedges, trapezoids, triangles, may be used.

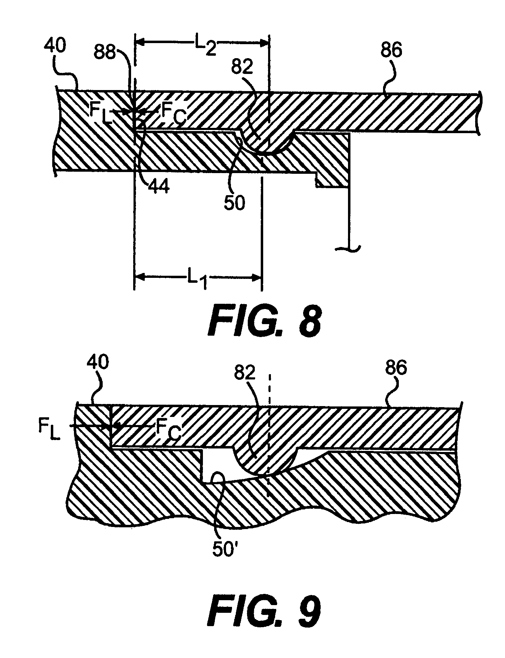

It is preferred that the linker, capsule units, and cap units be configured and dimensioned along with the snap-fit elements to provide a compressively abutting contact between the linker band 40, particularly band side surfaces 42, 44, and wall ends 78 and 88 of capsule unit 70 and cap unit 80, respectively. As best seen in Fig. 8, which is an enlarged view of detail A of Fig. 7, the distance Li between ridge 82 and the capsule wall end 88 may be slightly larger than the distance L2 between groove 50 and band side 44 of linker 10. As such, upon engagement between the capsule wall end 88 and band side 44, the ridge 82 is not "fully seated" in groove 50. But as a consequence of the resiliency of cap end 86 an axial force tending to fully seat ridge 82 results in a force Fc exerted by a capsule wall and 88 against a linker band side 44 and a similar force FL opposing this force. This compressively abutting cap contact may provide increased dosage form integrity, because it may eliminate any gap between the raised band 40 and the capsule end wall 88 which might otherwise establish a groove, edge, or other surface inconsistency that might increase a tendency to disengage the capsule unit from the linker. Also, the compressive abutting contact may minimize the chance for radial movement between the capsule unit and the linker and provide a relatively smooth surface to be established across the contact therebetween, potentially facilitating easier swallowing.

Still further, the compressive abutting contact provided by band 40 might provide a further barrier as a mechanical seal against unwanted drug substance leakage from the capsule compartments or the linker. This would be in addition to the seal provided by the contact between the snap-fit elements on the linker and the capsule compartments and/or closure caps. Also, the components of the multicompartment dosage forms can be configured to have a slight interference fit between the outer surface of the linker component and the inner surfaces of the capsule and/or cap units, to provide yet another barrier against leakage, while still providing ease of assembly.

Other means for providing a compressive abutting contact between the capsule and cap units against the linker band are contemplated including the use of different cross-sectional geometries of the grooves and/or ridges, as stated previously. Such a variation is shown in Fig. 9 where a wedge-shaped groove 50' is used instead of a circular cross-section. Again, it is contemplated that the one skilled in the art given this configuration and dimensions the linkers, capsules and/or cap units and associated snap-fit elements may be adjusted to achieve the desired compressive abutting contact function.

Each of the capsule compartments, closure caps, linkers, and linker parts may be made of a transitional polymer and may comprise the same or different polymer. A transitional polymer is a polymer that changes shape, form, or structure within a gastro-intestinal environment, e.g., dispersible, dissolvable, disintegrable, breachable, swellable, partially or completely soluble, fracturable, or otherwise changeable when exposed to stomach pH and/or in intestine pH. Suitable polymers include: polyvinyl alcohol (PVA), natural polymers (such as polysaccharides like pullulan, carrageenan, xanthan, chitosan or agar gums), polyethylene glycols (PEG), polyethylene oxides (PEO), mixtures of PEGS and PEOS, hydroxypropylmethylcellulose (HPMC), methylcellulose, hydroxyethylcellulose, hydroxyethyl methylcellulose, hydroxypropylcellulose, methacrylic acid copolymer (such as Eudragit E^{™}, Eudragit L^{™} and/or Eudragit S ^{™}), ammonium methacrylate copolymers (such as Eudragit RL^{™} and/or Eudragit RS ^{™}), carboxymethylcellulose, povidone (polyvinyl pyrrolidone), polyglycolysed glycerides (such as Gelucire 44/14^{™}, Gelucire 50/02^{™}, Gelucire 50/13^{™} and Gelucire 53/10^{™}), carboxyvinyl polymers (such as Carbopols^{™}), polyoxyethylene- polyoxypropylene copolymers (such as Poloxamer 188^{™}), and acrylic and/or methacrylic acid-based polymers. The Eudragit^{™} polymers discussed above for example are extrudable and may for example be plasticised with e.g. triethyl citrate, or glyceryl monostearate.

Preferred polymers are orally ingestible polymers and include hydroxypropyl methylcellulose acetate succinate (HPMC-AS), polyvinyl alcohol, hydroxypropyl methyl cellulose, and other cellulose-based polymers. Preferred polymers also include polymer materials which preferentially dissolve or disintegrate at different points in the digestive tract. Such polymers include the known acrylic and/or methacrylic acid-based polymers which are transitional in intestinal fluids, e.g. the Eudragit series of commercially available polymers. Examples of these include Eudragit E^{™}, such as Eudragit E 100 ^{™} or Eudragit 4135F^{™}, which preferentially dissolves in the more acid pH of the stomach, or enteric polymers such as Eudragit L^{™} and/or Eudragit S^{™} which preferentially dissolve in the more alkaline pH of the intestine, and preferred polymers also include polymers which dissolve slowly, e.g. at a predetermined rate in the digestive tract, such as Eudragit RL^{™} e.g. Eudragit RL 100 ^{™}, and/or Eudragit RS^{™} e.g. Eudragit R100 ^{™}, and/or blends of such Eudragit ^{™} polymers.

The polymers may include other substances to modify their properties and to adapt them to various applications, including, for example, the following general classes of substances: surfactants, such as Polysorbate 80^{™}, sodium lauryl sulphate, and Polyoxyl 40 ^{™} hydrogenated castor oil; absorption enhancers, such as Labrasol ^{™}, Transcutol^{™}; glidants, such as stearyl alcohol, talc, magnesium stearate, silicon dioxide, amorphous silicic acid, fumed silica, Simeticone^{™}; plasticizers, such as triethyl citrate, acetyl triethyl citrate, tributyl citrate, acetyl tributyl citrate, glyceryl monostearate, diethyl phthalate, dibutyl phthalate, propylene glycol, triacetin and castor oil; substances for release modification, such as ethyl cellulose and cellulose acetate phthalate; disintegrants, such as sodium starch glycollate, croscarmellose sodium, crospovidone (cross-linked polyvinyl pyrrolodone), coloring agents, flavoring agents and sweetening agents.

It is contemplated that in addition and/or as an alternative to the full or partial circumferential beads and grooves, a linker or a linker part may be connected to a capsule compartment, a closure cap, and/or another linker part via a threaded screw- type connection. It is also contemplated that if such a threaded screw-type connection is utilized, a first one of the capsule compartment, closure cap, linker, or linker part may include an external threaded element and a second one of the capsule compartment, closure cap, linker, or linker part may include an internal threaded element configured to be complementary to and engagable with the external threaded element. It is further contemplated that if such a threaded screw-type connection is utilized, the material from which the capsule compartment, closure cap, linker, or linker part may be strengthened via material re-work, additives to the polymer, and/or increased tolerances with respect to the mold or injection process as is known in the art.

Other embodiments of the invention will be apparent to those skilled in the art from consideration of the specification and practice of the invention disclosed herein. It is intended that the specification and examples be considered as exemplary only, with a true scope of the invention being indicated by the following claims.

## Claims

1. A linker (10) for connecting with one or more dosage form units from a group including capsule compartments and closure caps, the linker including a drug substance and a snap-fit element (48, 50) **characterized by**:
a solid drug substance in tablet form (12), the tablet (12) having a longitudinal axis and being substantially cylindrical with opposed axial end faces (16, 18);
a jacket (30) radially confining the tablet, the jacket (30) having an outer wall (32) with longitudinal ends (34, 36), at least one of the jacket longitudinal ends (34, 36) being open for dispensing the drug substance from the respective end face (16, 18), wherein the outer wall (32) includes the snap-fit element (48, 50) located adjacent at least one jacket longitudinal end (34, 36).

2. The linker (10) as in claim 1, **characterized by** the jacket (30) having a closed end wall (38) adjacent to one or the other of said jacket longitudinal ends (34, 36) for substantially blocking tablet drug substance(12) from dispensing from the respective tablet axial end face (16, 18).

3. The linker (10) as in claim 1, **characterized by** a raised circumferential band (40) on the jacket outer wall (32) between the jacket longitudinal ends (34, 36), the band (40) including a side surface (42, 44) configured for axial abutting contact with the dosage form unit.

4. The linker (10) as in claim 1, **characterized in that** at least one of the tablet end faces (16, 18) is exposed and configured to extend axially beyond a respective jacket longitudinal end (34, 36).

5. The linker (10) as in claim 1, **characterized in that** the tablet (12) has a recessed land (24, 26) around the perimeter of at least one tablet axial end faces (16, 18), and wherein the jacket (30) includes inwardly directed outer wall end edges (35, 37) that engage the recessed land (24, 26).

6. The linker (10) as in claim 1 , **characterized in that** the tablet (12) has two or more cylindrical parts (20, 22) in axially abutting contact, wherein the parts are comprised of different drug substances having different compositions and/or different release characteristics.

7. The linker (10) as in claim 4, **characterized in that** the linker (10) connects two dosage form units (70, 80), wherein the raised band (40) has two opposed side surfaces (42, 44) for abuttingly contacting the two units (70, 80), and wherein the raised band (40) has a top surface configured and dimensioned to transition between outer surfaces of the two dosage form units (70, 80).

8. The linker (10) as in claim 1, wherein the jacket (30) comprises a material selected from the group consisting of hydroxypropyl cellulose, hydroxypropyl methylcellulose acetate succinate, polyvinyl alcohol, hydroxypropyl methyl cellulose, and acrylic or methacrylic acid-based polymers.

9. A multicomponent dosage form comprising:
a linker unit (10) as claimed in claim 1 including
a solid drug substance in tablet form (12), the tablet having a longitudinal axis (14) and being substantially cylindrical with opposed longitudinal end faces (16, 18);
a jacket (30) radially confining the tablet (12), the jacket (30) having an outer wall (32) with longitudinal ends (34, 36) and a raised circumferential band (40) between the jacket longitudinal ends (34, 36), and wherein the outer wall (32) further includes a snap-fit element (48, 50) located adjacent at least one longitudinal end (34, 36), the element being selected from circumferential grooves (50), groove segments, ridges (72) and ridge segments; and
at least one dosage form unit selected from the group including capsule compartments (80) and closure caps (70), the dosage form unit having an open end connected to the one jacket longitudinal end, the unit having a snap-fit element complementary to and configured to engage the snap-fit element adjacent the one jacket longitudinal end.

10. The multicomponent dosage form as in claim 9, **characterized in that** the jacket (30) also has a closed end wall (38).

11. The multicomponent dosage form of claim 10, **characterized in that** the linker (10), jacket (30) and closed wall (38) each comprise a material selected from the group consisting of hydroxypropyl cellulose, hydroxypropyl methylcellulose acetate succinate, polyvinyl alcohol, hydroxypropyl methyl cellulose, and acrylic or methacrylic acid-based polymers.

12. The multicomponent dosage form of claim 9, **characterized in that** the dosage form unit (70, 80) has an inner surface (74, 84) proximate the open end, the dosage form unit complementary snap-fit element (72, 82) being located on a dosage unit inner surface (74, 84), and wherein the snap-fit element (48, 50) of the jacket (30) and the complementary snap-fit element (72, 82) of the dosage form unit are configured and longitudinally spaced to provide compressive abutting contact between the jacket raised circumferential band (40) and the open end of the dosage form unit, when the respective snap-fit (48, 50) and complementary snap-fit elements (72, 82) are engaged.

13. The multicomponent dosage form of claim 9, **characterized in that** the raised circumferential band (40) has a side configured (42, 44) for contacting the open end of the dosage form unit, and a longitudinal spacing between the raised band side and the snap-fit element (48, 50) on the longitudinal jacket end (34, 36) is less than a longitudinal spacing between the dosage form unit open end and the complementary snap-fit element (72, 82) on the inner wall (74, 84) of the connected dosage form unit.

14. The multicomponent dosage form of claim 9, **characterized in that** the linker unit (10) snap-fit element (48, 50) is a circumferential groove and the dosage form unit complementary snap-fit element (72, 82) is a circumferential ridge.

15. The multicomponent dosage form of claim 14, wherein the circumferential groove has generally curved (50) or wedge-shaped axial cross-section (50').

## Patentansprüche

1. Verbinder (10) zum Verbinden mit einer oder mehreren Darreichungsformeinheiten aus einer Gruppe, die Kapselabteile und Verschlusskappen enthält, wobei der Verbinder eine Arzneimittelsubstanz und ein Schnappelement (48, 50) aufweist, **gekennzeichnet durch**:
eine feste Arzneimittelsubstanz in Tablettenform (12), wobei die Tablette (12) eine Längsachse aufweist und im Wesentlichen zylindrisch ist, mit einander gegenüberliegenden axialen Stirnflächen (16, 18);
einen Mantel (30), der die Tablette radial begrenzt, wobei der Mantel (30) eine Außenwand (32) mit Längsenden (34, 36) aufweist, wobei mindestens eines der Längsenden (34, 36) des Mantels offen ist, um die Arzneimittelsubstanz von der jeweiligen Stirnfläche (16, 18) abzugeben, wobei die Außenwand (32) das Schnappelement (48, 50) aufweist, das mindestens einem Längsende (34, 36) des Mantels benachbart angeordnet ist.

2. Verbinder (10) nach Anspruch 1, **dadurch gekennzeichnet, dass** der Mantel (30) eine geschlossene Endwand (38) aufweist, die dem einen oder dem anderen von den Längsenden (34, 36) des Mantels benachbart ist, um ein Abgeben von Arzneimittelsubstanz der Tablette (12) von der jeweiligen axialen Stirnfläche (16, 18) der Tablette im Wesentlichen zu blockieren.

3. Verbinder (10) nach Anspruch 1, **gekennzeichnet durch** ein erhabenes Umfangsband (40) an der Außenwand (32) des Mantels zwischen den Längsenden (34, 36) des Mantels, wobei das Band (40) eine Seitenfläche (42, 44) aufweist, die für einen Kontakt mit der Darreichungsformeinheit **durch** axiale Anlage ausgebildet ist.

4. Verbinder (10) nach Anspruch 1, **dadurch gekennzeichnet, dass** mindestens eine der Stirnflächen (16, 18) der Tablette freiliegend ist und dafür ausgebildet ist, sich axial über ein jeweiliges Längsende (34, 36) des Mantels hinaus zu erstrecken.

5. Verbinder (10) nach Anspruch 1, **dadurch gekennzeichnet, dass** die Tablette (12) eine zurückgesetzte Fläche (24, 26) um den Umfang mindestens einer axialen Stirnfläche (16, 18) der Tablette herum aufweist, und wobei der Mantel (30) nach innen gerichtete Endränder (35, 37) der Außenwand aufweist, welche mit der zurückgesetzten Fläche (24, 26) in Eingriff gelangen.

6. Verbinder (10) nach Anspruch 1, **dadurch gekennzeichnet, dass** die Tablette (12) zwei oder mehr zylindrische Teile (20, 22) aufweist, die sich in Kontakt durch axiale Anlage befinden, wobei die Teile aus verschiedenen Arzneimittelsubstanzen mit unterschiedlichen Zusammensetzungen und/oder unterschiedlichen Freisetzungsmerkmalen bestehen.

7. Verbinder (10) nach Anspruch 4, **dadurch gekennzeichnet, dass** der Verbinder (10) zwei Darreichungsformeinheiten (70, 80) verbindet, wobei das erhabene Band (40) zwei einander gegenüberliegende Seitenflächen (42, 44) aufweist, die dazu bestimmt sind, mit den zwei Einheiten (70, 80) anliegend in Kontakt zu kommen, und wobei das erhabene Band (40) eine obere Fläche aufweist, die dafür ausgebildet und bemessen ist, einen Übergang zwischen äußeren Flächen der zwei Darreichungsformeinheiten (70, 80) zu bilden.

8. Verbinder (10) nach Anspruch 1, wobei der Mantel (30) ein Material umfasst, das aus der Gruppe ausgewählt ist, welche aus Hydroxypropylcellulose, Hydroxypropylmethylcelluloseacetatsuccinat, Polyvinylalkohol, Hydroxypropylmethylcellulose und Polymeren auf Acryl- oder Methacrylsäurebasis besteht.

9. Mehrkomponenten-Darreichungsform, welche umfasst:
eine Verbindungseinheit (10) nach Anspruch 1, welche aufweist:
eine feste Arzneimittelsubstanz in Tablettenform (12), wobei die Tablette eine Längsachse (14) aufweist und im Wesentlichen zylindrisch ist, mit einander gegenüberliegenden axialen Stirnflächen (16, 18);
einen Mantel (30), der die Tablette (12) radial begrenzt, wobei der Mantel (30) eine Außenwand (32) mit Längsenden (34, 36) und einem erhabenen Umfangsband (40) zwischen den Längsenden (34, 36) des Mantels aufweist, und wobei die Außenwand (32) ferner ein Schnappelement (48, 50) aufweist, das mindestens einem Längsende (34, 36) benachbart angeordnet ist, wobei das Element aus Umfangsnuten (50), Nutensegmenten, Rippen (72) und Rippensegmenten ausgewählt ist; und
mindestens eine Darreichungsformeinheit, die aus der Gruppe ausgewählt ist, die Kapselabteile (80) und Verschlusskappen (70) enthält, wobei die Darreichungsformeinheit ein offenes Ende aufweist, das mit dem einen Längsende des Mantels verbunden ist, wobei die Einheit ein Schnappelement aufweist, das komplementär zu dem Schnappelement ist, welches dem einen Längsende des Mantels benachbart ist, und dafür ausgebildet ist, mit diesem Schnappelement in Eingriff zu gelangen.

10. Mehrkomponenten-Darreichungsform nach Anspruch 9, **dadurch gekennzeichnet, dass** der Mantel (30) auch eine geschlossene Endwand (38) aufweist.

11. Mehrkomponenten-Darreichungsform nach Anspruch 10, **dadurch gekennzeichnet, dass** der Verbinder (10), der Mantel (30) und die geschlossene Wand (38) jeweils ein Material umfassen, das aus der Gruppe ausgewählt ist, welche aus Hydroxypropylcellulose, Hydroxypropylmethylcelluloseacetatsuccinat, Polyvinylalkohol, Hydroxypropylmethylcellulose und Polymeren auf Acryl- oder Methacrylsäurebasis besteht.

12. Mehrkomponenten-Darreichungsform nach Anspruch 9, **dadurch gekennzeichnet, dass** die Darreichungsformeinheit (70, 80) eine Innenfläche (74, 84) nahe dem offenen Ende aufweist, wobei das komplementäre Schnappelement (72, 82) der Darreichungsformeinheit an einer Innenfläche (74, 84) der Darreichungsformeinheit angeordnet ist, und wobei das Schnappelement (48, 50) des Mantels (30) und das komplementäre Schnappelement (72, 82) der Darreichungsformeinheit so ausgebildet und in Längsrichtung beabstandet sind, dass sie einen Druckkontakt durch Anlage zwischen dem erhabenen Umfangsband (40) des Mantels und dem offenen Ende der Darreichungsformeinheit gewährleisten, wenn sich das jeweilige Schnappelement (48, 50) und komplementäre Schnappelement (72, 82) in Eingriff befinden.

13. Mehrkomponenten-Darreichungsform nach Anspruch 9, **dadurch gekennzeichnet, dass** das erhabene Umfangsband (40) eine Seite (42, 44) aufweist, die dafür ausgebildet ist, mit dem offenen Ende der Darreichungsformeinheit in Kontakt zu kommen, und ein Längsabstand zwischen der Seite des erhabenen Bandes und dem Schnappelement (48, 50) an dem Längsende (34, 36) des Mantels kleiner als ein Längsabstand zwischen dem offenen Ende der Darreichungsformeinheit und dem komplementären Schnappelement (72, 82) an der Innenwand (74, 84) der verbundenen Darreichungsformeinheit ist.

14. Mehrkomponenten-Darreichungsform nach Anspruch 9, **dadurch gekennzeichnet, dass** das Schnappelement (48, 50) der Verbindungseinheit (10) eine Umfangsnut ist und das komplementäre Schnappelement (72, 82) der Darreichungsformeinheit eine Umfangsrippe ist.

15. Mehrkomponenten-Darreichungsform nach Anspruch 14, wobei die Umfangsnut im Wesentlichen einen gekrümmten (50) oder keilförmigen axialen Querschnitt (50') aufweist.

## Revendications

1. Dispositif de liaison (10) pour relier une ou plusieurs unités de formes posologiques unitaires d'un groupe comprenant des compartiments à capsule et des capuchons de fermeture, le dispositif de liaison comprenant une substance médicamenteuse et un élément ajusté par pression (48, 50), **caractérisé par** :
une substance médicamenteuse solide sous la forme d'un comprimé (12), le comprimé (12) ayant un axe longitudinal et étant sensiblement cylindrique avec des faces d'extrémité axiale opposées (16, 18) ;
une chemise (30) confinant radialement le comprimé, la chemise (30) ayant une paroi externe (32) avec des extrémités longitudinales (34, 36), au moins l'une des extrémités longitudinales (34, 36) de la chemise étant ouverte pour distribuer la substance médicamenteuse par la face d'extrémité respective (16, 18),
dans lequel la paroi externe (32) comprend l'élément ajusté par pression (48, 50) situé adjacent au moins à une extrémité longitudinale (34, 36) de la chemise.

2. Dispositif de liaison (10) selon la revendication 1, **caractérisé en ce que** la chemise (30) présente une paroi d'extrémité fermée (38) adjacente à l'une ou à l'autre desdites extrémités longitudinales (34, 36) de la chemise pour bloquer sensiblement toute distribution de la substance médicamenteuse (12) du comprimé par la face d'extrémité axiale respective (16, 18) du comprimé.

3. Dispositif de liaison (10) selon la revendication 1, **caractérisé par** une bande circonférentielle dressée (40) sur la paroi externe (32) de la chemise entre les extrémités longitudinales (34, 36) de la chemise, la bande (40) comprenant une surface latérale (42, 44) configurée pour venir en contact d'aboutement axial avec l'unité de forme posologique.

4. Dispositif de liaison (10) selon la revendication 1, **caractérisé en ce qu'**au moins l'une des faces d'extrémité (16, 18) du comprimé est exposée et configurée pour s'étendre axialement au-delà d'une extrémité longitudinale respective (34, 36) de la chemise.

5. Dispositif de liaison (10) selon la revendication 1, **caractérisé en ce que** le comprimé (12) a une zone évidée (24, 26) autour du périmètre d'au moins une face d'extrémité axiale (16, 18) du comprimé et dans lequel la chemise (30) comprend des bords d'extrémité de parois externes (35, 37) dirigés vers l'intérieur, qui s'engagent sur la zone évidée (24, 26).

6. Dispositif de liaison (10) selon la revendication 1, **caractérisé en ce que** le comprimé (12) a deux parties cylindriques (20, 22) ou plus en contact d'aboutement axial, dans lequel les parties sont constituées de différentes substances médicamenteuses ayant différentes compositions et/ou différentes caractéristiques de libération.

7. Dispositif de liaison (10) selon la revendication 4, **caractérisé en ce que** le dispositif de liaison (10) raccorde deux unités de formes posologiques (70, 80), dans lequel la bande dressée (40) a deux surfaces latérales opposées (42, 44) pour venir en contact d'aboutement avec les deux unités (70, 80) et dans lequel la bande dressée (40) a une surface supérieure configurée et dimensionnée pour faire la transition entre des surfaces externes des deux unités de formes posologiques (70, 80).

8. Dispositif de liaison (10) selon la revendication 1, dans lequel la chemise (30) comprend un matériau choisi dans le groupe constitué de l'hydroxypropylcellulose, de l'hydroxypropyl-méthylcellulose, de l'acétate succinate d'hydroxypropylméthylcellulose, de l'alcool polyvinylique, de l'hydroxypropylméthylcellulose et de polymères à base d'acide acrylique ou méthacrylique.

9. Forme posologique multicomposant comprenant :
une unité de liaison (10) selon la revendication 1, comprenant :
une substance médicamenteuse solide sous forme de comprimé (12), le comprimé ayant un axe longitudinal (14) et étant sensiblement cylindrique avec des faces d'extrémité longitudinales opposées (16, 18) ;
une chemise (30) confinant radialement le comprimé (12), la chemise (30) ayant une paroi externe (32) avec des extrémités longitudinales (34, 36) et une bande circonférentielle dressée (40) entre les extrémités longitudinales (34, 36) de la chemise, et dans laquelle la paroi externe (32) comprend en outre un élément d'ajustement sous pression (48, 50) situé adjacent à au moins une extrémité longitudinale (34, 36), l'élément étant choisi entre des rainures circonférentielles (50), des segments de rainures, des nervures (72) et des segments de nervures ; et
au moins une unité de forme posologique choisie dans le groupe comprenant des compartiments de capsules (80) et des capuchons de fermeture (70), l'unité de forme posologique ayant une extrémité ouverte raccordée à une extrémité longitudinale de la chemise, l'unité ayant un élément ajusté sous pression complémentaire de l'élément ajusté sous pression adjacent à la une extrémité longitudinale de la chemise et configuré pour s'y engager.

10. Forme posologique multicomposant selon la revendication 9, **caractérisée en ce que** la chemise (30) présente également une paroi d'extrémité fermée (38).

11. Forme posologique multicomposant selon la revendication 10, **caractérisée en ce que** le dispositif de liaison (10), la chemise (30) et la paroi fermée (38) comprennent chacun un matériau choisi dans le groupe constitué de l'hydropropylcellulose, de l'acétate succinate d'hydroxypropylméthylcellulose, de l'alcool polyvinylique, de l'hydroxypropylméthylcellulose et de polymères à base d'acide acrylique ou méthacrylique.

12. Forme posologique multicomposant selon la revendication 9, **caractérisée en ce que** l'unité de forme posologique (70, 80) a une surface interne (74, 84) proche de l'extrémité ouverte, l'élément ajusté sous pression (72, 82) complémentaire de l'unité de forme posologique étant situé sur une surface interne (74, 84) de l'unité posologique, et dans lequel l'élément ajusté sous pression (48, 50) de la chemise (30) et l'élément complémentaire ajusté sous pression (72, 82) de l'unité de forme posologique sont configurés et espacés longitudinalement pour assurer une surface d'aboutement compressif entre la bande circonférentielle dressée (40) de la chemise et l'extrémité ouverte de l'unité de forme posologique lorsque l'élément ajusté sous pression respectif (48, 50) et les éléments ajustés sous pression complémentaires (72, 82) sont engagés.

13. Forme posologique multicomposant selon la revendication 9, **caractérisée en ce que** la bande circonférentielle dressée (40) a un côté configuré (42, 44) pour venir en contact avec l'extrémité ouverte de l'unité de forme posologique et un espacement longitudinal entre le côté de la bande dressée et l'élément ajusté sous pression (48, 50) sur l'extrémité longitudinale (34, 36) de la chemise est inférieur à un espacement longitudinal entre l'extrémité ouverte de l'unité de forme posologique et l'élément ajusté sous pression complémentaire (72, 82) sur la paroi interne (74, 84) de l'unité de forme posologique reliée.

14. Forme posologique multicomposant selon la revendication 9, **caractérisée en ce que** l'élément ajusté sous pression (48, 50) de l'unité de liaison (10) est une rainure circonférentielle et l'élément d'ajustement sous pression complémentaire (72, 82) de l'unité de forme posologique est une nervure circonférentielle.

15. Forme posologique multicomposant selon la revendication 14, dans laquelle la rainure circonférentielle a une section transversale axiale (50') généralement incurvée (50) ou en forme de coin.
